# EUROPEAN PATENT SPECIFICATION

(11) **EP 1 199 052 B1**
(45) Date of publication and mention of the grant of the patent: **18.12.2013**
(21) Application number: 01130605.7
(22) Date of filing: 04.03.1998
(51) Int. Cl.: A61F 2/90, A61F 2/915, A61F 2/954, A61F 2/958, A61M 25/10

(54) **Stent and catheter assembly and method for treating bifurcations**
Vorrichtung zum Einführen eines Stents zur Behandlung von Verzweigungen
Appareil pour l'introduction d'un stent pour traiter des bifurcations

(30) Priority: 13.08.1997 US 910857
(43) Date of publication of application: 24.04.2002
(62) Divisional of application: 98301613.0
(73) Proprietor: Abbott Cardiovascular Systems Inc., Santa Clara, CA 95054 (US)
(72) Inventor: Wilson, Stan W., Missoula, Montana 59802 (US); Mauch, Kevin M., Windsor, California 95492 (US)
(74) Representative: Boult Wade Tennant

(56) References cited:
- WO-A-96/29955
- WO-A-97/41803
- WO-A1-98/19628
- FR-A- 2 737 969
- US-A- 5 211 683
- US-A- 5 456 712
- US-A- 5 653 743

## Description

### BACKGROUND OF THE INVENTION

### Field of the Invention

The invention relates to stent deployment assemblies for use at a bifurcation and, more particularly, to stents for repairing bifurcations, the aorto-ostium, and bifurcated blood vessels that are diseased.

### Prior Art

Stents conventionally repair blood vessels that are diseased and generally are hollow and cylindrical in shape, having terminal ends that are generally perpendicular to its longitudinal axis. In use, the conventional stent is positioned at the diseased area of a vessel and, after placement, the stent provides an unobstructed pathway for blood flow.

Repair of vessels that are diseased at a bifurcation is particularly challenging as the stent must overlay the entire diseased area at the bifurcation, yet not itself compromise blood flow. Therefore, the stent, without compromising blood flow, must overlay the entire circumference of the ostium to a diseased portion and extend to a point within and beyond the diseased portion. When the stent does not overlay the entire circumference of the ostium to the diseased portion, the stent fails to completely repair the bifurcated vessel. When the stent overlays the entire circumference of the ostium to the diseased portion, yet extends into the junction comprising the bifurcation, the diseased area is repaired, but blood flow may be compromised in other portions of the bifurcation. Unapposed stent elements may promote lumen compromise during neointimalization and healing, producing restenosis and requiring further procedures. Moreover, by extending into the junction comprising the bifurcation, the stent may block access to portions of the bifurcated vessel that require performance of further interventional procedures. Similar problems are encountered when a vessel is diseased at the point of its angled origin from the aorta, as in the ostium of a right coronary artery or a vein graft. In this circumstance, a stent overlying the entire circumference of the ostium extends back into the aorta, creating problems, including that of complicating catheter access to the vessel in any additional interventional procedures.

Conventional stents are designed to repair areas of blood vessels that are removed from the sites of bifurcations and, because a conventional stent generally terminates at right angles to its longitudinal axis, the use of conventional stents in the region of a vessel bifurcation may result in blocking the flow of blood through a side branch or may fail to repair the bifurcation to the full extent necessary. A conventional stent might be placed so that a portion of the stent extends into the pathway of blood flow to a side branch of the bifurcation or so that it extends so far as to completely cover the path of blood flow in a side branch. A conventional stent alternatively might be placed proximal to, but not entirely overlaying, the circumference of the ostium to the diseased portion. Such positioning of a conventional stent results in a bifurcation that is not completely repaired. The only conceivable situation in which a conventional stent having right-angled terminal ends could be placed where the entire circumference of the ostium is repaired without compromising blood flow, is where the bifurcation is formed of right angles. In such scenarios, extremely precise positioning of the conventional stent is required. This extremely precise positioning of the conventional stent may result in the right-angled terminal ends of the conventional stent overlying the entire circumference of the ostium to the diseased portion without extending into a side branch, thereby completely repairing the right-angled bifurcation.

To circumvent or overcome the problems and limitations associated with conventional stents in the context of repairing diseased bifurcated vessels, a stent that consistently overlays the entire circumference of the ostium to a diseased portion, yet does not extend into the junction comprising the bifurcation, may be employed. Such a stent has the advantage of completely repairing the vessel at the bifurcation without obstructing blood flow in other portions of the bifurcation. In addition, such a stent allows access to all portions of the bifurcated vessel, should further interventional treatment be necessary. In a situation involving disease located at the origin of an angulated aorto-ostial vessel, such a stent would have the advantage of completely repairing the vessel at the origin without protruding into the aorta and without complicating repeat access.

In addition to the problems encountered by using the prior art stents to treat bifurcations, the delivery platform for implanting such stents has presented numerous problems. For example, a conventional stent is implanted in the main vessel so that a portion of the stent is across the side branch, so that stenting of the side branch must occur through the main-vessel stent struts. In this method, commonly referred to in the art as the "monoclonal antibody" approach, the main-vessel stent struts must be spread apart to form an opening to the side-branch vessel and then a catheter with a stent is delivered through the opening. The "cell" or the portion of the main-vessel stent struts to be spread apart must be randomly and blindly selected by re-crossing the deployed stent with a wire. A drawback with this approach is that there is no way to determine or guarantee that the main-vessel stent struts are oriented properly with respect to the side branch or that the appropriate cell has been selected by the wire for dilatation. The aperture created often does not provide a clear opening and creates significant distortion in the surrounding stent struts. Thus, there is no way to tell if the main-vessel stent struts have been properly oriented and spread apart to provide a clear opening for stenting the side-branch vessel.

In another prior art method for treating bifurcated vessels, commonly referred to as the "Culotte technique," the side-branch vessel is first stented so that the stent protrudes into the main vessel. A dilatation is then performed in the main vessel to open and stretch the stent struts extending across the lumen from the side-branch vessel. Thereafter, the main-vessel stent is implanted so that its proximal end overlaps with the side-branch vessel. One of the drawbacks of this approach is that the orientation of the stent elements protruding from the side-branch vessel into the main vessel is completely random. Furthermore, the deployed stent must be re-crossed with a wire blindly, and then arbitrarily selecting a particular stent cell. When dilating the main vessel, the stretching of the stent struts therefore is random, leaving the possibility of restricted access, incomplete lumen dilatation, and major stent distortion.

In another prior art device and method of implanting stents, a "T" stent procedure includes implanting a stent in the side-branch ostium of the bifurcation followed by stenting the main vessel across the side-branch ostium. In another prior art procedure, known as "kissing" stents, a stent is implanted in the main vessel with a side-branch stent partially extending into the main vessel creating a double-barreled lumen of the two stents in the main vessel distal to the bifurcation. Another prior art approach includes a so-called "trouser legs and seat" approach, which includes implanting three stents, one stent in the side-branch vessel, a second stent in a distal portion of the main vessel, and a third stent, or a proximal stent, in the main vessel just proximal to the bifurcation.

All of the foregoing stent deployment assemblies suffer from the same problems and limitations. Typically, there are uncovered segments of the intimal surface of the main vessel and side-branch vessels between the segments that are stented. An uncovered flap or fold in the intima or plaque will invite a "snowplow" effect, representing a substantial risk for sub-acute thrombosis, and the increased risk of restenosis. Further, when portions of the stent are left un-apposed within the lumen, the risk of sub-acute thrombosis or restenosis again is increased. The prior art stents and delivery assemblies for treating bifurcations are difficult to use, making successful placement of the stents nearly impossible. Further, even where placement has been successful, the side-branch vessel can be "jailed" or covered so that there is impaired access to the stented area for subsequent intervention.

In an attempt to overcome these difficulties there is disclosed in WO 97141803, which is prior art under Art. 54(3) EPC, an expandable bifurcated stent comprising a proximal end and a distal end in communication with one another. The proximal end comprises a primary passageway and the distal end comprises a pair of secondary passageways. The stent is expandable from a first, contracted position to a second, expanded position upon the application of a radially outward force exerted on the stent. Each of the primary passageway and the secondary passageway has a porous surface. A method for production of a bifurcated stent is also described comprising the step of connecting a first stent section to a second stent section, the first stent section having an end thereof adapted for connection to an opening disposed along the length of a second stent section.

In FR-A-2,737,969 there is described a vascular endoprosthesis, generally shaped as a radially extensible cylinder, and used for maintaining a body canal, particularly an artery, in an expanded state. The endoprosthesis is characterised in that the length of the generators of the expanded endoprosthesis, as measured in relation to a predetermined transversal plane, is not constant, so that it can be implanted at a fork in a body canal.

In WO 96/29965 there is described a tubular prosthesis which is introduced to a region of an aneurysm and expanded. A particular advantage of using the prosthesis is that the diameter will be supported by eversion along its entire length. Additionally, the presence of the multiple eversion helps assure good radially outward contact between the prosthesis and the blood vessel wall along its length.

In US-A-5,456,712 there is described a graft and stent assembly securable to a first sidewall of a blood vessel having an arteriotomy defined therein, with the blood vessel having a flow of blood therein. The assembly includes a graft which has an orifice. The graft further has an end portion which is positionable within the blood vessel and substantially adjacent to a portion of the first sidewall of the blood vessel which substantially surrounds the arteriotomy. The assembly further includes a stent which has a second sidewall. The stent is positionable within the blood vessel and in contact with the end portion of the graft so as to secure the end portion of the graft between the first sidewall of the blood vessel and the stent. The orifice of the graft is positioned relative to the second sidewall of the stent so that the flow of blood must pass through the second sidewall of the stent in order to advance through the orifice of the graft.

In addition to the problems that are encountered in treating disease involving bifurcations for vessel origins, difficulty also is encountered in treating disease that is confined to a vessel segment which extends very close to a distal branch point or bifurcation that is not diseased and that does not require treatment. In such circumstances, it may be difficult or impossible to very precisely place a stent to cover the distal segment, but not to extend into the ostium of the distal side-branch.

Preferred embodiments of the present invention offer a solution to the above problems.

References to distal and proximal herein shall mean as follows: the proximal direction is moving away from or out of the patient and the distal direction is moving toward or into the patient. These definitions will apply with reference to body lumens and apparatus, such as catheters, guide wires, and stents.

### SUMMARY OF THE INVENTION

Preferred embodiments of the present invention provide improved stent assemblies for repairing a main vessel and a side-branch vessel forming a bifurcation, without compromising blood flow in other portions of the bifurcation, thereby allowing access to all portions of the bifurcated vessels should further interventional treatment be necessary.

According to the present invention there is provided an assembly for treating a bifurcation having the features set out in claim 1.

The assembly includes a first, side branch catheter and a proximal-angled stent mounted on a balloon portion of the first, side-branch catheter for implanting in a side-branch vessel adjacent to a bifurcation between the side-branch vessel and a main-vessel. The proximal-angled stent comprises a cylindrical member which can have substantially any outer wall surface typical of conventional stents which have been used, for example, in the coronary arteries. The cylindrical member of the proximal-angled stent has a distal end forming a first plane section that is substantially transverse to a longitudinal axis of the stent The proximal end of the stent forms a second plane section that is at an acute angle measured relative to the longitudinal axis of the stent. The acute angle is selected to approximately coincide with the angle formed by the intersection of the side-branch vessel and the main vessel, so that after deployment no portion of the stented area mine side-branch vessel is left uncovered, and no portion of the proximal-angled stent extends into the main vessel.

In addition the assembly further comprises a second, main-vessel catheter and a main-vessel stent mounted on a balloon portion of the second, main-vessel catheter for implanting in the main vessel adjacent to the bifurcation. The main-vessel stent has a cylindrical member with distal and proximal ends and an outer wall surface therebetween, which typically can be similar to the outer wall surface of stents that have been used in the coronary arteries. An aperture is formed in the outer wall surface of the main-vessel stent and is sized and positioned on the outer wall surface so that when the stent is implanted in the main vessel, the aperture is aligned with the side-branch vessel and the proximal-angled stent in the side-branch vessel, providing unrestricted blood flow from the main vessel through to the side-branch vessel. Deployment of the angled and apertured stents is accomplished by a —— stent delivery system adapted specifically for treating bifurcated vessels.

In one example of a stent delivery system for implanting the proximal-angled stent, a side-branch catheter is provided in which a tracking guide wire lumen extends within at least a portion of the side-branch catheter, being designed to be either an over-the-wire or rapid exchange-type catheter. An expandable member is disposed at the distal end of the side-branch catheter. A tracking guide wire is provided for slidable movement within the tracking guide wire lumen. A positioning guide wire lumen is associated with the catheter and the expandable member, such that a portion of the positioning guide wire lumen is on the outer surface of the catheter and it approaches the proximal end of the outer surface of the expandable member. A stent-positioning guide is provided for slidable movement within the positioning lumen. The proximal ends of the tracking and stent-positioning guide wires extend out of the patient and can be manipulated simultaneously so that the distal end of the stent-positioning guide wire is advanced in the main vessel distal to a side-branch vessel, and the distal end of the tracking guide wire is advanced into the side-branch vessel distal to the side-branch vessel target area. In one arrangement, the stent-positioning guide wire lumen includes an angulated section so that the stent-positioning guide wire advanced in the main vessel distal to the side-branch vessel results in rotation, causing the proximal-angled stent to assume the correct position in the side-branch vessel. The positioning lumen functions to orient the stent-positioning guide wine to rotate or to torque the side-branch catheter so as to properly align and position the proximal-angled stent in the side-branch vessel.

The side-branch catheter assembly is capable of delivering the proximal-angled stent, mounted on the expandable member, in the side-branch vessel, The side-branch catheter also could be configured for delivering a self-expanding proximal-angled stent.

In one arrangement the delivery system further includes a main-vessel catheter for delivering a stent in the main vessel after the side-branch vessel has been stented. The main-vessel catheter includes a tracking guide wire lumen, extending through at least a portion thereof, which is adapted for receiving a tracking guide wire for slidable movement therein. An expandable member is positioned near the main-vessel catheter distal end for delivering and implanting a main-vessel (apertured) stent in the main vessel. The main-vessel stent includes an aperture on its outer surface which aligns with the side-branch vessel. A positioning guide wire lumen is associated with the expandable member, and is sized for slidably receiving the stent-positioning guide wire. The stent-positioning guide wire slides within the positioning guide wire lumen to orient the expandable member so that it is positioned adjacent to, but not in, the side-branch vessel with the aperture of the stent facing the side-branch ostium.

In one arrangement, both the side-branch catheter and main-vessel catheter assemblies include the so-called rapid exchange catheter features, which allow one catheter to be exchanged easily for another catheter while the tracking and positioning guide wires remain positioned, respectively, in the side-branch vessel and the main vessel. In an alternate arrangement, both catheters may be of the "over-the-wire" type.

In addition to the above there is also described a method for delivering the proximal-angled and the main-vessel (apertured) stents in the bifurcated vessel. In a preferred arrangement of the side-branch catheter system (i.e., side-branch catheter and proximal-angled stent), the distal end of the tracking guide wire is advanced into the side-branch vessel and distally of the target area. The side-branch catheter then is advanced along the tracking guide wire until the distal end of the catheter is just proximal of the side-branch. The distal end of the integrated, stent-positioning guide wire then is advanced by the physician, who pushes the guide wire from outside the body. The distal end of the stent-positioning wire travels through the positioning guide wire lumen, passes close to the proximal end of the proximal-angled stent and expandable member, and then exits the lumen. The wire is advanced in the main vessel until the distal end is distal to the side-branch vessel. The catheter then is advanced into the side branch until resistance is felt, owing to the stent-positioning guide wire pushing up against the ostium of the side-branch vessel, which causes the proximal-angled stent to rotate into position and to arrest its advancement at the ostium. Thereafter, the proximal-angled stent, mounted on the expandable member, is aligned across the side-branch vessel target area and the angled proximal end of the stent is aligned at the intersection of the side-branch vessel and the main vessel (the ostium of the side-branch vessel) so that the stent completely covers the target area in the side-branch vessel, but does not extend into the main vessel, where it may block blood flow. The expandable member is expanded thereby expanding and implanting the proximal-angled stent in the side-branch vessel. The positioning wire prevents forward movement of the expandable member and proximal-angled stent during inflation. Thereafter, the expandable member is deflated and the side-branch catheter assembly is withdrawn from, the patient in a known rapid-exchange manner. In this arrangement, the side-branch catheter is designed so that both the side-branch tracking guide wire and main-vessel positioning guide wire can be left in the vessel in which each respectively was deployed in the event sequential or simultaneous high pressure balloon inflation is required in a vessel in order to complete the stenting procedure. In other words, the integrated positioning wire can be unzipped from the proximal 100 cm of the catheter thereby allowing it to act as a rapid exchange wire. Preferably, high pressure balloons are inflated simultaneously in the main vessel stent and proximal-angled stent, in order to avoid deforming one stent by unopposed balloon inflation within the other one. This additional step of high pressure balloon inflation is a matter of physician preference. A further advantage of this arrangement is that by refraining from advancing the integrated stent-positioning wire out of catheter until the catheter distal end is near the target area avoids wire wrapping, which can be encountered in an arrangement using two non-integrated guide wires. When using this method, the side-branch vessel can be stented without the need for stenting the main vessel.

In an aorto-ostial application of the side-branch catheter assembly (side-branch catheter together with a proximal angulated stent), the positioning wire is advanced into the aortic root while the tracking wire is advanced into the right coronary or vein graft, the angulated origin of which is to be stented. After the proximal-angled stent mounted on the expanding member is advanced, it is aligned across the target area and the angled proximal end of the stent is aligned at the ostium.

In the event the main vessel is to be stented (with the stent placed across the bifurcation site), the proximal end of the main-vessel guide wire is inserted into the distal end of the guide wire lumen of the main-vessel catheter. The side-branch wire would be removed from the side branch at this time. The main-vessel catheter then would be advanced into the body until the catheter is within 1 cm or so of the target site. The distal end of the second (integrated, stent-positioning) guide wire, which resides in the main-vessel catheter during delivery to the main vessel, then is advanced by having the physician push the positioning wire from outside the body. The distal end of the stent-positioning wire travels through the positioning guide wire lumen and passes underneath the proximal half of the stent until it exits at the site of the stent aperture or at the location of a designated stent cell where an aperture can be formed. The catheter then is advanced distally until resistance is felt, owing to the stent-positioning guide wire pushing up against the ostium of the side-branch vessel, which indicate that the stent aperture correctly is facing the side-branch vessel ostium and is aligned with the proximal end of the proximal-angled stent. Thereafter, the expandable member on the main-vessel catheter is inflated, thereby expanding the main-vessel stent into contact with the main vessel and implanting it, with the aperture in the stent providing an unobstructed flow path for the blood from the main vessel through to the side-branch vessel. The expandable member then is deflated and the main-vessel catheter is removed from the body. The main-vessel catheter is designed so that both the main-vessel guide wire and side-branch wire can be left in the vessels in which each respectively was deployed in the event sequential or simultaneous high pressure balloon inflation is required in a vessel in order to complete the stenting procedure. The presence of the stent-positioning wire in the stent aperture permits catheter access through this aperture into the side-branch vessel for balloon inflation to smooth out the aperture in the main-vessel stent. This additional step is a matter of physician preference.

When using this method, the main vessel can be stented without the need for stenting the side-branch vessel. An advantage of this arrangement: is that a maj or side branch, not diseased and not requiring treatment, that exits off from a main vessel that does require stenting, may be protected by the positioning wire while the main vessel is stented. If "snowplowing" compromise or closure of the side-branch vessel should occur in the course of the main-vessel stenting, then access already is established and guaranteed for stenting of the side-branch vessel over the wire already in place in the manner described above. This will allow confident stetting of a main vessel segment containing a major side branch, In this usage, additional stenting of the side branch will be required only if compromise or occlusion of the side branch occurs.

Other features and advantages of the present invention will become apparent from the following detailed description, taken in conjunction with the accompanying drawings, which illustrate, by way of example, the principles of the invention.

### BRIEF DESCRIPTION OF THE DRAWINGS

FIGURE 1 is an elevational view of a bifurcation in which a prior art "T" stent is in a side-branch ostium followed by the stenting of the main vessel across the branch ostium.
FIG. 2 is an elevational view of a bifurcation in which "touching" prior art stents are depicted in which one stent is implanted in the side branch, a second stent is implanted in a proximal portion of the main vessel next to the branch stent, with interrupted placement of a third stent, implanted more distally in the main vessel.
FIG. 3 is an elevational view of a bifurcation depicting "kissing" stents where a portion of one stent is implanted in both the side-branch and the main vessel and adjacent to a second stent implanted in the main vessel, creating a double-barreled lumen in the main vessel distal to the bifurcation.
FIG. 4 is an elevational view of a prior art "trouser legs and seat" stenting approach depicting one stent implanted in the side-branch vessel, a second stent implanted in a proximal portion of the main vessel, and a close deployment of a third stent distal to the bifurcation leaving a small gap between the three stents of an uncovered luminal area.
FIG. 5A is a perspective view of a stent having an angled proximal end.
FIG. 5B is a side elevational view of the proximal-angled stent of FIG. 5A depicting the distal end being transverse to the longitudinal axis of the stent, and the proximal end at an angle of less than 90°.
FIG. 5C is an elevational view of a bifurcation in which a prior art stent is implanted in the side-branch vessel.
FIG. 5D is an elevational view of a bifurcation in which a prior art stent is implanted in the side-branch vessel, with the proximal end of the stent extending into the main vessel.
FIG. 5E is an elevational view of a bifurcation in which the proximal-angled stent depicted in FIGS. 5A and 5B, is implanted in the side-branch vessel.
FIG. 6A is a perspective view depicting a main-vessel stent in which an aperture is formed on the outer surface of at least a portion of the stent.
FIG. 6B is a side elevational view of the main-vessel stent of FIG. 6A.
FIG. 7A is an elevational view, partially in section, of a side-branch catheter assembly depicting the distal end of the catheter with the expandable member and the second guide wire lumen attached thereto, for receiving the integrated stent-positioning guide wire, while the tracking guide wire is received by the main guide wire lumen.
FIG. 7B is an elevational view, partially in section, of the catheter assembly of FIG. 7A, in which the stent positioning guide wire is advanced out of the catheter.
FIG. 8 is an elevational view, partially in section, of a side-branch catheter assembly depicting an expandable balloon having an angled proximal portion corresponding to the angle of the proximal-angled stent.
FIG. 9A is an elevational view of a bifurcated vessel in which a side-branch tracking guide wire has been advanced into a side-branch vessel, with the stent-positioning guide wire remaining within the catheter until the catheter assembly is just proximal to the side-branch vessel.
FIG. 9B is an elevational view of a bifurcation in which a side-branch tracking guide wire has been advanced through the patient's vascular system into a side branch, and a stent-positioning guide wire has been advanced through the patient's vascular system and into the main vessel distal to the ostium of the side-branch vessel.
FIG. 10A is an elevational view of a bifurcation in which the side-branch catheter assembly has been advanced in the patient's vasculature so that the proximal-angled stent mounted on the expandable member is positioned in the target area of the side-branch vessel.
FIG. 10B is an elevational view of the side-branch catheter assembly of FIG. 10A in which the proximal-angled stent has been expanded by the balloon portion of the catheter in the side-branch vessel.
FIGS. 11A-11D are partial elevational views in which the side-branch catheter assembly of FIG. 10A is used to implant the proximal-angled stent in the side-branch vessel where the proximal-angled stent is rotated to be properly aligned for implanting in the vessel.
FIGS. 12A-12C depict an elevational view, partially in section, of a main-vessel catheter assembly in which the main vessel stent has an aperture on its outer surface.
FIGS. 12D-12F depict an elevational view, partially in section, of the main-vessel catheter of FIGS. 12A-12C with a ramp to help orient and advance the guide wire through the aperture in the main-vessel stent.
FIGS. 12G-12I depict an elevational view, partially in section, of an alternative to the main-vessel catheter of FIGS. 12A-12C in which the guide wire lumen is angled to pass under the stent and exit through the stent aperture.
FIGS. 12J-12L depict an elevational view, partially in section, of an alternative to the main-vessel catheter of FIGS. 12A-12C in which a portion of the guide wire lumen passes under the sent.
FIGS. 13A-13E are elevational views, partially in section, depicting the main-vessel catheter assembly of FIG. 12A and the main-vessel stent in which two guide wires are used to correctly position the main vessel stent so that the aperture in the stent is aligned with the side-branch vessel.
FIG. 14 is an elevational view of a bifurcated vessel in which the proximal-angled stent is implanted in the side-branch vessel and a main vessel stent is implanted in the main vessel.

### DETAILED DESCRIPTION OF THE PREFERRED EMBODIMENTS

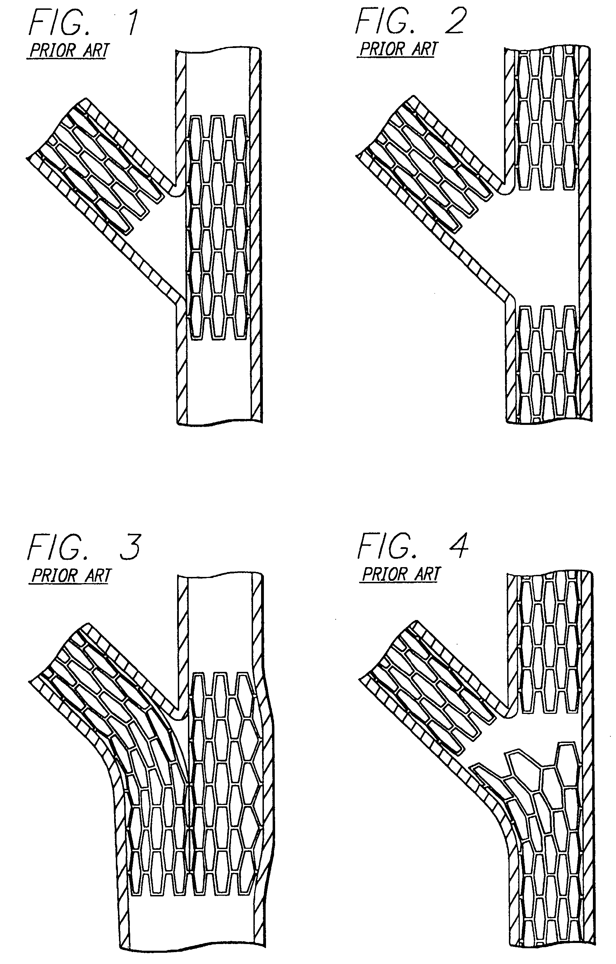

The present invention is directed to an assembly for treating bifurcations in, for example, the coronary arteries, veins, arteries, and other vessels in the body. Prior art attempts at implanting intravascular stents in a bifurcation have proved less than satisfactory. For example, FIGS. 1-4 depict prior art devices which include multiple stents being implanted in both the main vessel and a side-branch vessel. In FIG. 1, a prior art "T" stent is implanted such that a first stent is implanted in the side branch near the ostium of the bifurcation, and a second stent is implanted in the main vessel, across the side-branch ostium. With this approach, portions of the side-branch vessel are left uncovered, and blood flow to the side-branch vessel must necessarily pass through the main-vessel stent, causing possible obstructions or thrombosis.

Referring to FIG. 2, three prior art stents are required to stent the bifurcation. In FIG. 3, the prior art method includes implanting two stents side by side, such that one stent extends into the side-branch vessel and the main vessel, and the second stent is implanted in the main vessel. This results in a double-barreled lumen which can present problems such as thrombosis, and turbulence in blood flow. Referring to the FIG. 4 prior art device, a first stent is implanted in the side-branch vessel, a second stent is implanted in a proximal portion of the main vessel, and a third stent is implanted distal to the bifurcation, thereby leaving a small gap between the stents and an uncovered luminal area.

All of the prior art devices depicted in FIGS. 1-4 have carious drawbacks.

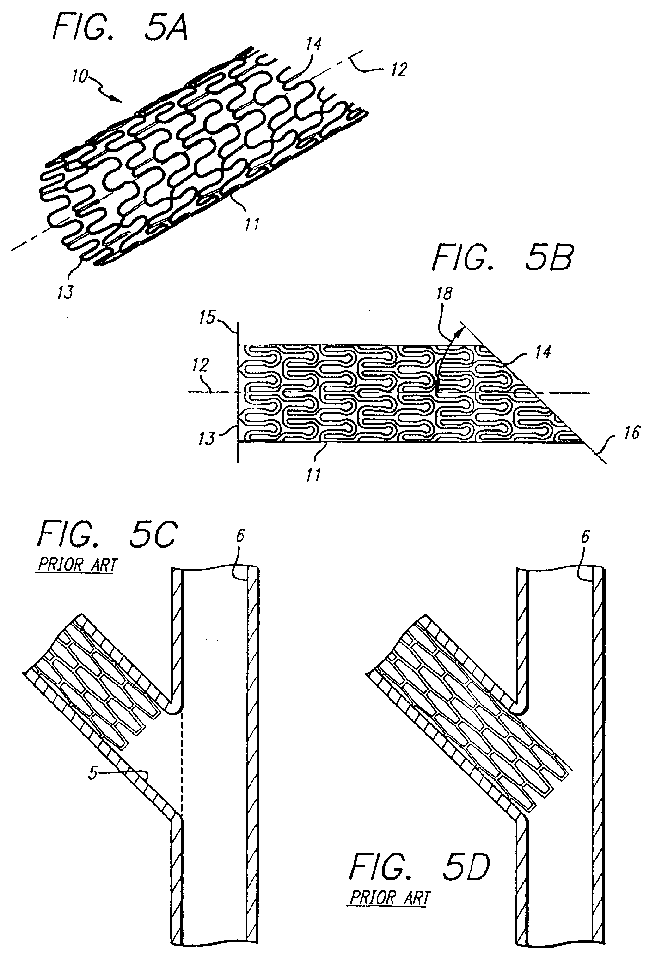

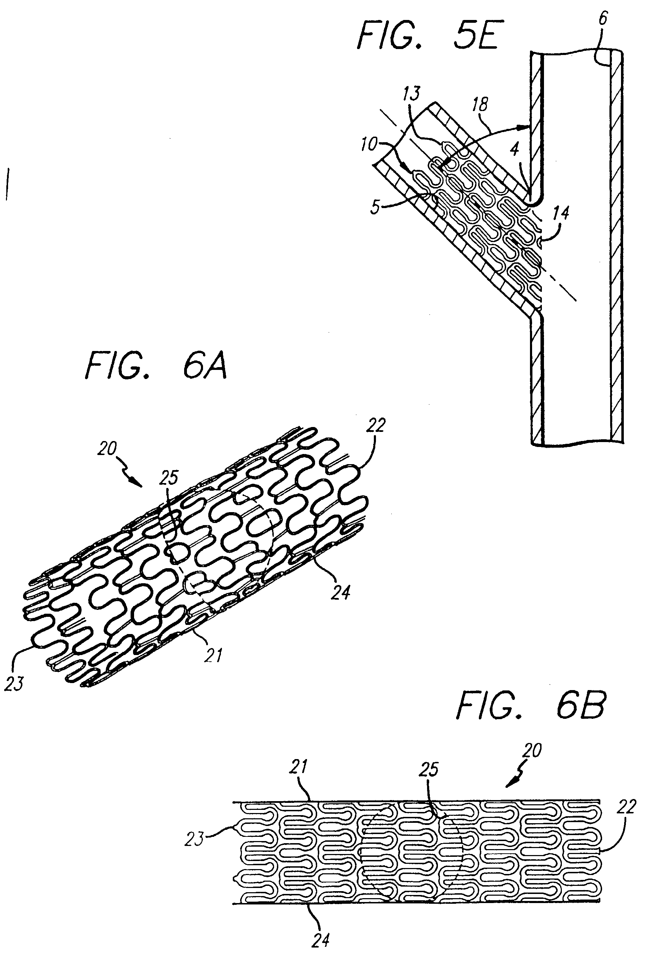

As depicted in FIGS. 5A, 5B and 5E, a proximal-angled stent 10 is configured for deployment in a side-branch vessel 5. The proximal-angled stent 10 includes a cylindrical member 11 having a longitudinal axis 12 which is an imaginary axis extending through the cylindrical member 11. A distal end 13 and a proximal end 14 define the length of the cylindrical member 11. A first plane section 15 is defined by a plane section through the distal end 13 of the cylindrical member, and second plane section 16 is defined by a plane section through the proximal end 14 of the cylindrical member. A second plane section 16 defines an acute angle 18, which is the angle between the second plane section 16 and the longitudinal axis 12.

In treating the side-branch vessel 5, if a prior art stent is used in which there is no acute angle at one end of the stent to match the angle of the bifurcation, a condition as depicted in FIGS. 5C and 5D will occur. That is, a stent deployed in the side-branch vessel 5 will leave a portion of the side-branch vessel exposed (FIG. 5C), or as depicted in 5D, a portion of the stent will extend into the main-vessel 6. As depicted in FIG. 5E, the proximal-angled stent 10 has an acute angle 18 that approximates the angle formed by the bifurcation 4 of the side-branch vessel 5 and the main-vessel 6. Thus, the acute angle 18 is intended to approximate the angle formed by the intersection of the side-branch 5 and the main-vessel 6. The angle between the side-branch vessel 5 and the main-vessel 6 will vary for each application, and for the present purposes, should be less than ninety degrees (90°). If there is a ninety degree (90°) angle between the side-branch vessel and the main vessel, a conventional stent having ends that are transverse to the stent longitudinal axis, would be suitable for stenting the side-branch vessel.

The proximal-angled stent can be implanted in the side-branch vessel to treat a number of angulated ostial lesions including, but not limited to, the following:
1. The ostium of a left anterior descending artery (LAD), where there is a circumflex or trifurcation vessel at less than ninety degrees (90 °) in its departure from the LAD.
2. The ostium of the circumflex artery or a trifurcation in a similar situation as number 1.
3. The ostium of a sizeable diagonal.
4. The LAD just distal to, but sparing, the origin of a diagonal.
5. The ostium of a circumflex marginal artery with an angulated take-off,
6. Disease in the circumflex artery just distal to a marginal take-off, but sparing that take-off.
7. The aorta-ostium of a right coronary artery with an angled take-off.
8. The origin of an angulated posterior descending artery.
9. The origin of an LV extension branch just at and beyond the crux, sparing the posterior descending artery.
10. The ostium of an angulated vein graft origin.
11. Any of many of the above locations in conjunction with involvement of the bifurcation and an alternate vessel.

The proximal-angled stent typically can be used as a solo device to treat the foregoing indications, or it can be used in conjunction with the main-vessel stent described herein for stenting the bifurcation.

As depicted in FIGS. 6A and 6B, the main-vessel stent 20 is configured for deployment in the main vessel 6. The main-vessel stent 20 includes a cylindrical member 21 having a distal end 22 and a proximal end 23. The main-vessel stent 20 includes an outer wall surface 24 which extends between a distal end 22 and a proximal end 23 and incorporates an aperture 25 on the outer wall surface 24. The aperture 25 is configured so that, upon expansion, it approximates the diameter of the expanded proximal end 14 of the proximal-angled stent 10. When the main-vessel stent 20 is implanted and expanded into contact with the main-vessel 6, the aperture 25 is aligned with the side-branch vessel 5 and the proximal end 14 of the proximal-angled stent 10, thereby providing an unrestricted blood flow path from the side-branch vessel to the main vessel. Unlike what has been available in the prior art, the main-vessel catheter allows selection and positioning of an aperture at the side-branch ostium. Further, it provides for the positioning of a guide wire during main-vessel stent deployment which can be used for additional intervention if necessary. In the prior art techniques, access to a side-branch is through a randomly selected stent element ("cell"), and is only possible after deployment of the stent. The precise positioning of aperture 25 in the stent illustrated in FIGS. 6A and 6B is optional and the aperture 25 could be positioned either closer to the proximal end or closer to the distal end of the main vessel stent 20.

The proximal-angled stent 10 and the main-vessel stent 20 can be formed from any of a number of materials including, but not limited to, stainless steel alloys, nickel-titanium (NiTi) alloys (the NiTi can be either a shape memory type or pseudoelastic), tantalum, tungsten, or any number of polymer materials. Such materials of manufacture are known in the art. Further, the proximal-angled stent 10 and the main-vessel stent 20 can have virtually any pattern known to prior art stents. In a preferred configuration, the proximal-angled stent 10 and the main-vessel stent 20 are formed from a stainless steel material and have a plurality of cylindrical elements connected by connecting members, wherein the cylindrical elements have an undulating or serpentine pattern. Such a stent is disclosed in U.S. Patent No. 5,514,154 and is manufactured and sold by Advanced Cardiovascular Systems, Inc., Santa Clara, California. The stent is sold under the tradename MultiLink® Stent. Such stents can be modified to include the features of the proximal-angled stent 10 (the angulation) and the main-vessel stent 20 (the aperture).

The proximal-angled stent 10 and the main-vessel stent 20 preferably are balloon-expandable devices that are mounted on a balloon portion of a catheter and crimped tightly onto the balloon to provide a low profile delivery diameter. After the catheter is positioned so that the stent and the balloon portion of the catheter are located either in the side-branch or the main vessel, the balloon is expanded, thereby expanding the stent beyond its elastic limit into contact with the vessel. Thereafler, the balloon is deflated and the balloon and catheter are withdrawn from the vessel, leaving the stent implanted. Deployment of the angled and main-vessel stents is accomplished by a - stent delivery system adapted specifically for treating bifurcated vessels. The proximal-angled stent and the main-vessel stent could be made to be either balloon expandable or self-expanding.

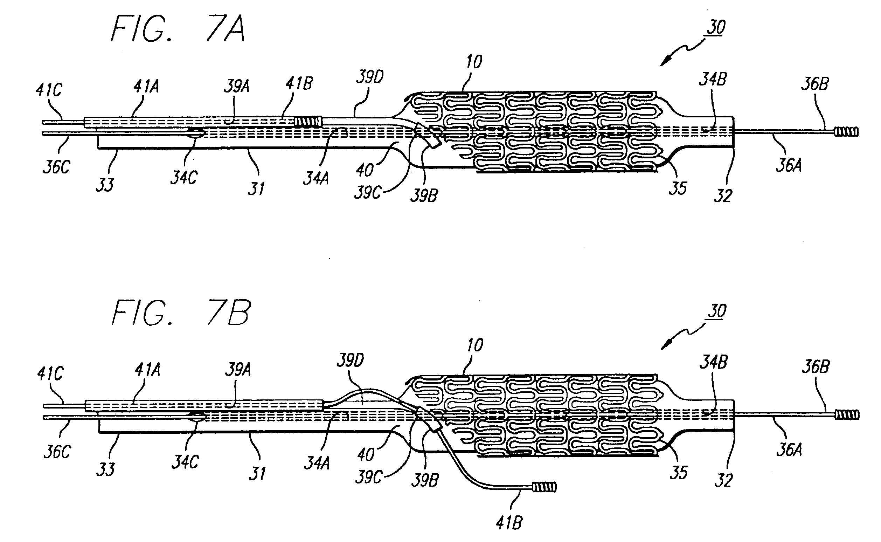

In one preferred arrangement for delivering the stents, as depicted in FIGS. 7A and 7B, the side-branch stent delivery assembly 30 is provided and includes side-branch catheter 31. The side-branch catheter includes a distal end 32 which is configured for delivery into the patient's vasculature and a proximal end 33 which remains outside the patient. A first guide wire lumen 34A extends through at least a portion of the side-branch catheter 31, depending on the type of catheter (e.g., rapid exchange, over-the-wire, etc.) desired for a particular application. The first guide wire lumen 34A preferably is defined by a distal end 34B and a side port 34C, which is typical of the so-called rapid-exchange-type catheters. Typically, a slit (not shown) extends from the side port 34C to just proximal of the balloon portion of the catheter, so that the catheter can be rapidly exchanged during a medical procedure, as is known.

The expandable member 35, which typically is a non-distensible balloon, has a first compressed diameter for delivery through the vascular system, and a second expanded diameter for implanting a stent. The expandable member 35 is positioned near the distal end 32, and in any event between the distal end 32 of the first catheter 31 and the side port 34C.

Referring to FIGS. 7A and 7B, a tracking guide wire 36A having a distal end 36B and a proximal end 36C extends through the first guide wire lumen 34A. The tracking guide wire 36A preferably is a stiff wire having a diameter of 0.356 mm (.014 inch), but can have a different diameter and stiffness as required for a particular application. An especially suitable guide wire can be one of those manufactured and sold under the tradenames Sport® and Ironman®, manufactured by Advanced Cardiovascular Systems, Inc., Santa Clara, California. The tracking guide wire 36A is sized for slidable movement within the first guide wire lumen 34A.

The side-branch delivery assembly 30 further includes a second guide wire lumen 39A, which is associated with the expandable member 35. The second guide wire lumen 39A includes an angle portion 39B and a straight portion 39C, and is firmly attached to an outer surface 40 of the catheter 31, at a point just proximal to the expandable member 35. An integrated stent-positioning guide wire 41 A is sized for slidable movement within the second guide wire lumen 39A. A slit 39D is formed in the lumen 39A near its distal end, so that the stiff guide wire 41A can bow outwardly as shown in FIG. 7B. The portion of the guide wire 41A that bows out of the slit 39D will limit the advancement of the catheter 31, as will be further described infra. The integrated stent-positioning guide wire 41A has a distal end 41 B, and a proximal end 41 C which extends out of the patient. Again, it is preferred that the integrated stent-positioning guide wire, 41 A be a fairly stiff wire as previously described, for the reasons set forth below in delivering and implanting the stents in the bifurcation.

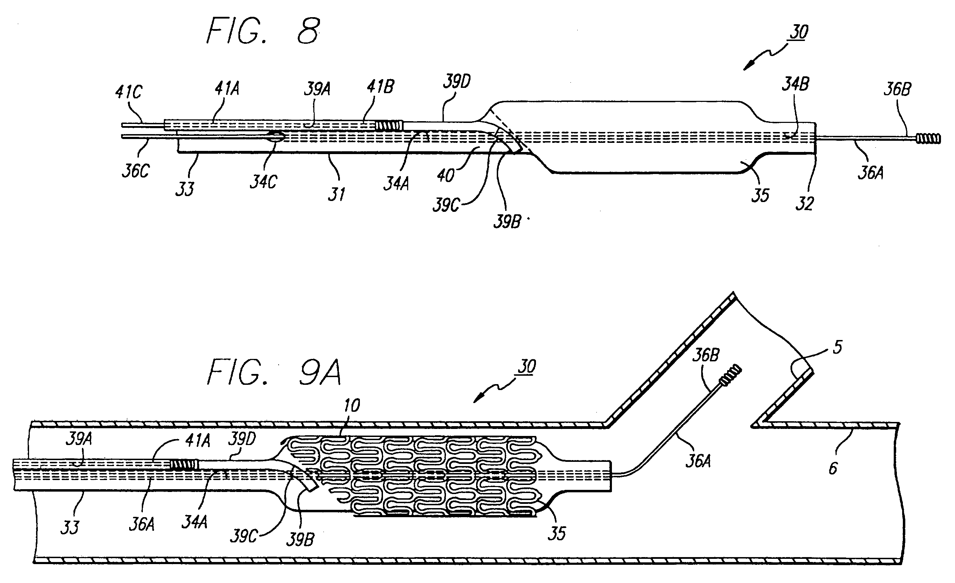

In an alternative arragement, the catheter 31 can have an angled expandable member 42 as depicted in FIG. 8. The proximal end of the expandable member is angled to coincide with the angle of the proximal-angled stent 10 (not shown in FIG. 8 for clarity). This arrangement is particularly useful in delivering the angled stent since the second guide wire lumen 39A, and its angled portion 39B, have the same angle as the stent and the proximal end of the expandable member.

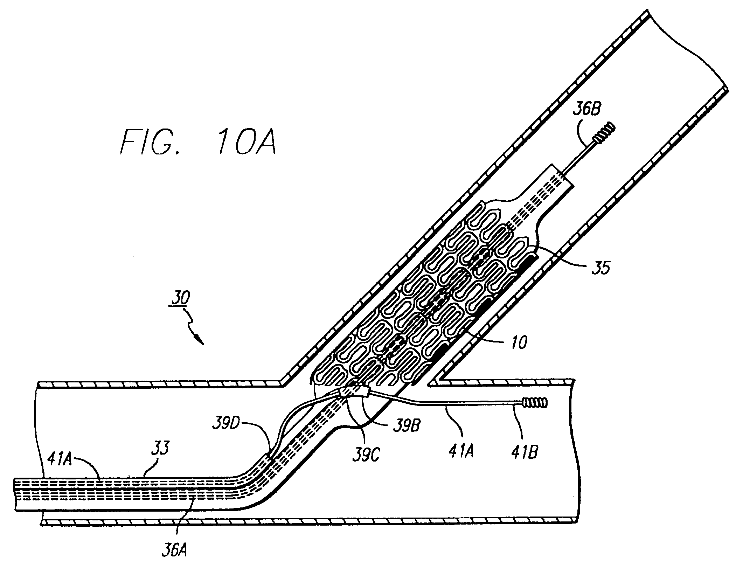

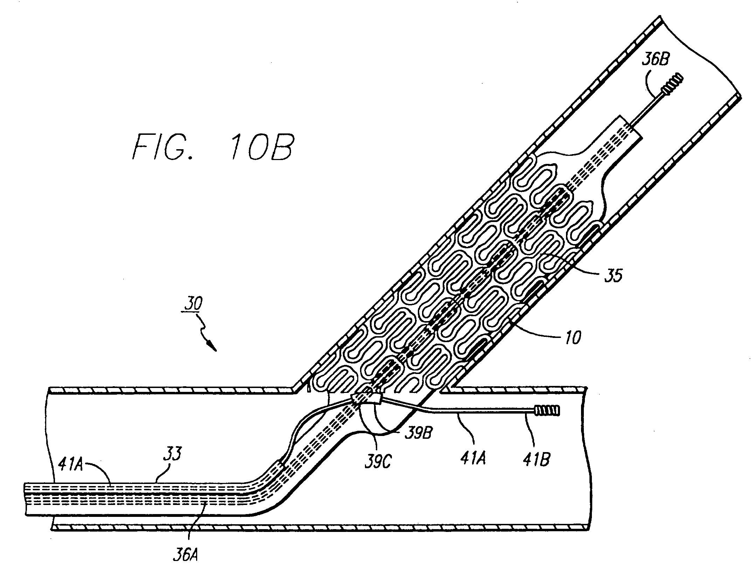

As depicted in FIGS. 9A-11D, the proximal-angled stent 10 is mounted on the side-branch catheter 31 and implanted in the side-branch vessel 5. The method of achieving proximal-angled stent implantation is as follows.

The proximal-angled stent 10 first is crimped tightly onto the expandable member 35 for low-profile delivery through the vascular system

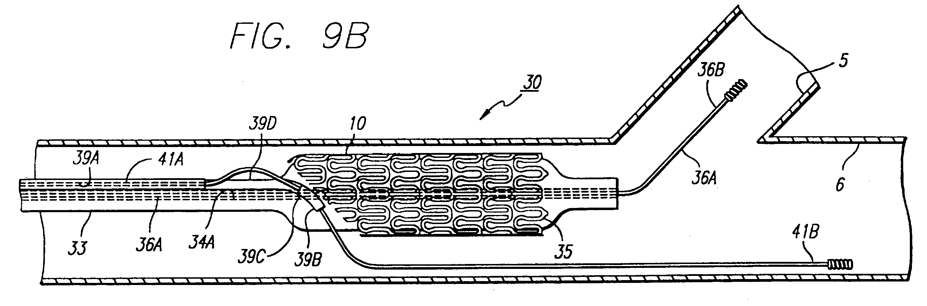

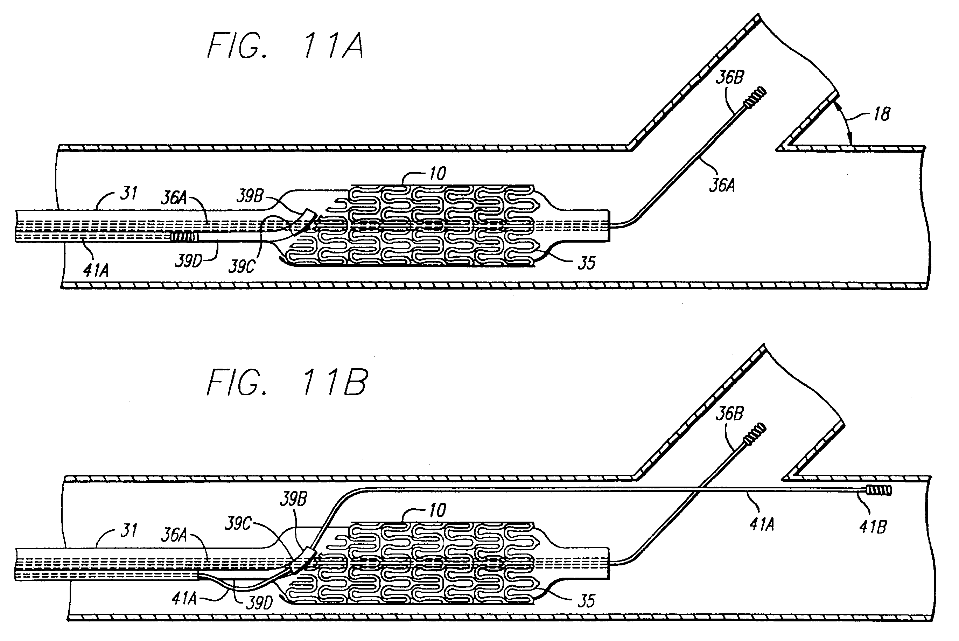

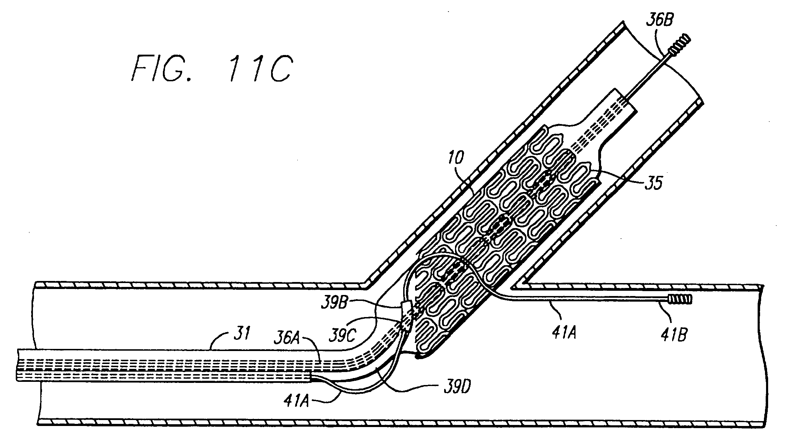

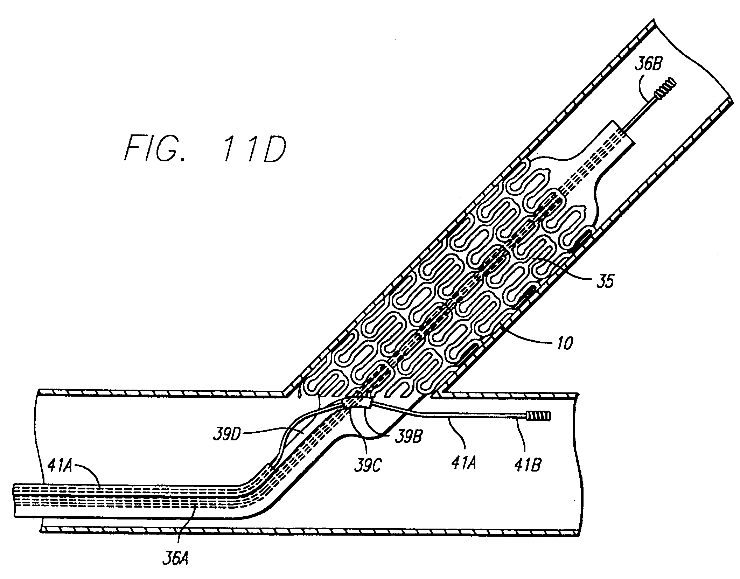

The distal end 36B of the guide wire 36A is advanced into the side-branch vessel 5 and distally of the target area, with the proximal end 36C remaining outside the patient. The side-branch catheter 31 then is advanced within a guiding catheter (not shown) along the tracking guide wire 36A until the distal end 32 of the catheter is just proximal (about 1 cm) of entering the side-branch vessel 5. Up to this point, the guide wire 41A resides in the second guide wire lumen 39A so that the distal end 41B of the wire preferably is near, but is not in, the angled portion 39B of the guide wire lumen 39A. This method of delivery prevents the two guide wires from wrapping around each other, the guide wire 41 A being protected by the catheter during delivery. The distal end 41B of the integrated stent positioning guide wire 41A then is advanced by having the physician push the proximal end 41 C from outside the patient's body. The distal end 41B of the integrated stent-positioning guide wire travels through the guide wire lumen 39A and the angled portion 39B and passes close to the proximal end 14 of the angled stent 10 and the expandable member 35 and exits at the angled portion 39B of the second guide wire lumen 39B. As the guide wire 41A is advanced into, through and out of the angled portion 39B, the stiffness of the wire causes it to bow outwardly through the slit 39D in the distal portion of the second guide wire lumen 39A. Thus, as can be seen, for example, in FIGS. 9B, 10A, 10B, and 11B-11D, the positioning guide wire 41A bows outwardly and, due to its stiffness, provides a bumper against the ostium of the side-branch vessel to assist in positioning and deploying the stents. The stent-positioning guide wire 41A is advanced in the main vessel until the distal end 41B is distal to the side-branch vessel 5. The catheter then is advanced into the side-branch vessel 5 until resistance is felt, caused by the stent-positioning guide wire 41 A pushing up against the ostium of the side-branch vessel 5. As previously described, the stent-positioning wire 41A is relatively stiff, as is the tracking guide wire 36A, so that each can properly orient the side-branch catheter 31 as it is advanced into the side-branch vessel 5. The angled portion 39B of the second guide wire lumen 39A is angled to assist in rotating the side-branch catheter 31 into proper position into the side-branch vessel 5. If the stent approaches the side-branch vessel 5 in the incorrect position, as depicted in FIGS. 11A-11B, the stent-positioning wire 41 A would be forced to make a very acute angle. The wire stiffness, however, prevents this from happening and causes the wire to assume the position of least stress. As is illustrated in FIGS. 11C-11D, to relieve this stress buildup, the wire 41A creates a torque on the angled portion 39B, causing the guide wire lumen 39A and the side-branch catheter 31, with the proximal-angled stent 10, to rotate into the correct position. Preferably, the slit 39D is formed on the outer surface 40 of the catheter 31 near the angled portion 39B, so that the stent-positioning guide wire 41A can bow outwardly out of the slit 39D, thereby increasing the ability to torque the catheter and the proximal-angled stent.

Thereafter, the proximal-angled stent 10 mounted on the expandable member 35 is aligned across the target area, and viewed under fluoroscopy, the acute angle 18 on the proximal end of the proximal-angled stent is aligned at the intersection of the side-branch vessel 5 and the main-vessel 6 (the ostium of the side-branch vessel) so that the proximal-angled stent completely covers the target area in the side-branch vessel 5, yet does not extend into the main-vessel 6, thereby compromising blood flow. The expandable member 35, which typically is a non-distensible balloon, is expanded by known methods, thereby expanding the proximal-angled stent into contact with the side-branch vessel 5, and thereby implanting the proximal-angled stent in the side-branch vessel. Thereafter, the expandable member 35 is deflated and the side-branch catheter assembly 31 is withdrawn from the patient's vasculature. The side-branch catheter 31 is designed so that both the tracking guide wire 36A and the stent-positioning guide wire 41 A can be left in the vessels in which each respectively has been deployed, in the event sequential or simultaneous high pressure balloon inflation be required in each of the vessels in order to complete the stenting procedure. In other words, the integrated positioning wire can be unzipped through the slit (not shown) from the proximal 100 cm of the catheter, thereby allowing it to act as a rapid exchange wire. Preferably, high pressure balloons are inflated simultaneously in the main-vessel stent and the proximal-angled stent, in order to avoid deforming one stent by unopposed balloon inflation occurring in the other one. This additional step is a matter of physician preference. Using this method, the side-branch vessel 5 can be stented without the need for stenting the main vessel, as shown in FIGS. 11A-11D.

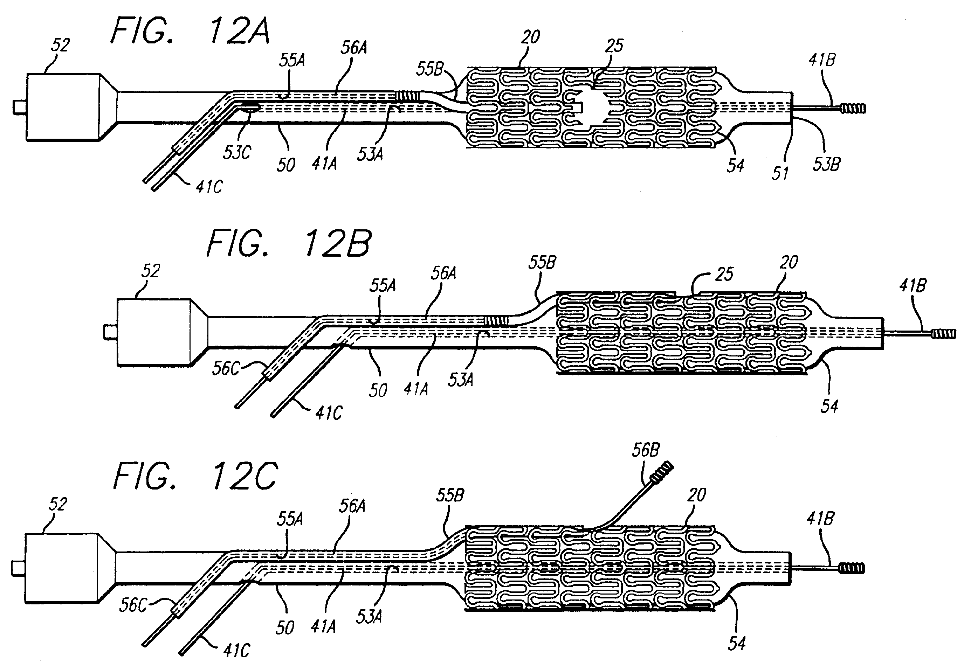

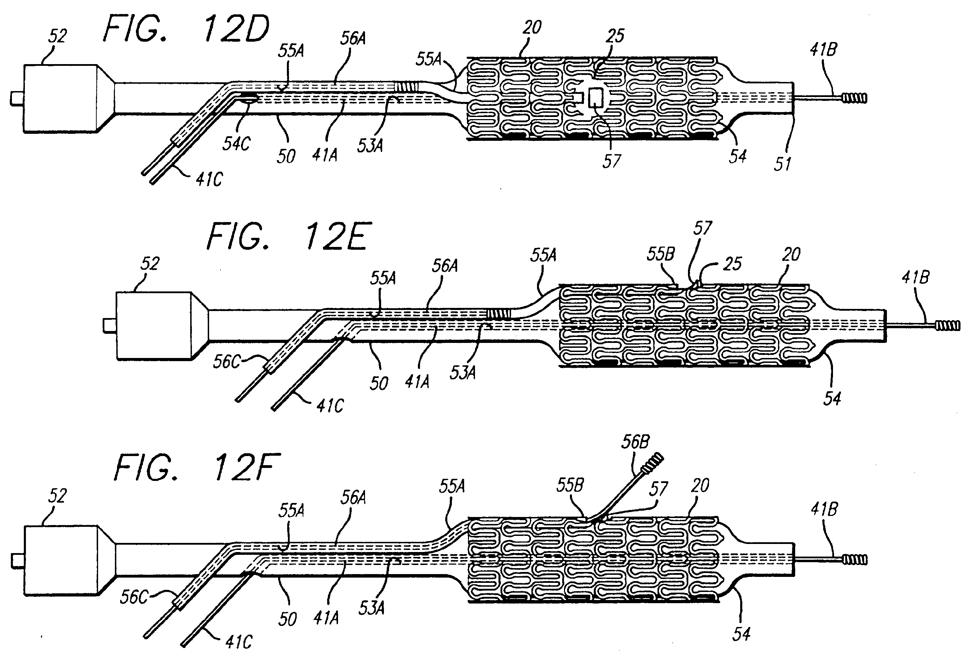

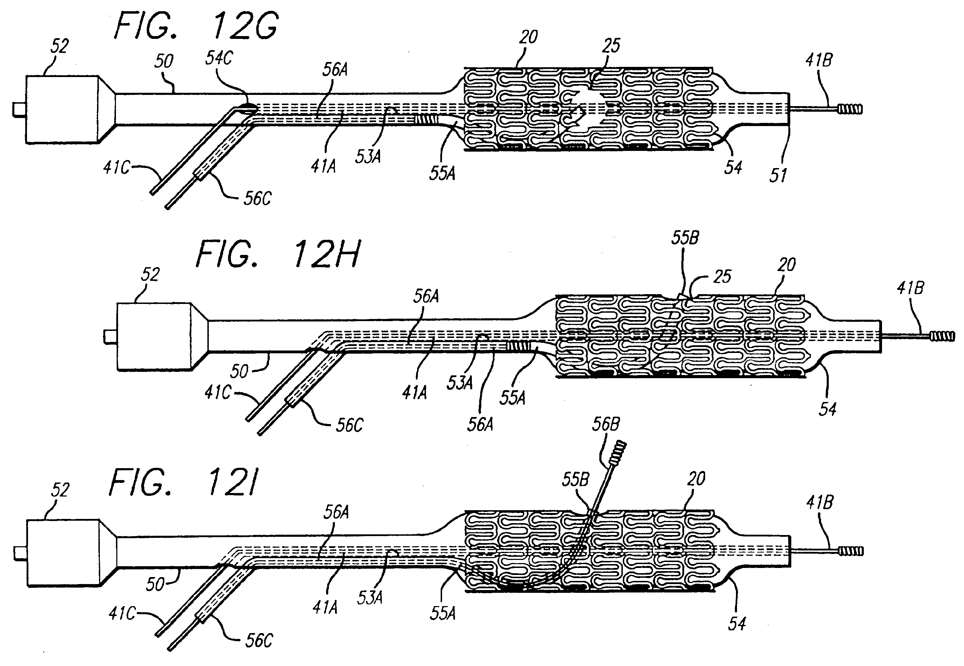

If necessary, the main-vessel 6 also can be stented after stenting the side-branch vessel. In that regard, a main-vessel catheter assembly 50 is provided for implanting the main-vessel stent 20, as depicted in FIGS. 12A to 13E. In one preferred arrangement, as shown in FIGS. 12A-12C, the main-vessel catheter 50 includes a distal end 51 which is configured for advancement within the patient's vasculature, and a proximal end 52 which remains outside the patient. The main-vessel catheter includes a guide wire lumen 53A having a distal end 53B and a side port 53C, which is proximal to the balloon portion of the catheter. The side port 53C is provided in a so-called rapid-exchange catheter system which includes a slit (not shown) as is known in the art. An expandable member 54 is located near the distal end 51 of the main-vessel catheter 50. Typically, the expandable member 54 is a non-distensible balloon of the type known in the art for delivering and expanding stents.

The positioning guide wire lumen 55A is positioned partly on the catheter shaft and partly on the expandable member 54, and is configured for slidably receiving the integrated stent-positioning guide wire 56A. Prior to stent delivery, the integrated stent-positioning guide wire 56A resides in the positioning guide wire lumen 55A and only during stent delivery is it then advanced into and through the angled portion 55B of the lumen,

Other preferred arrangements for implanting the main-vessel stent 20 in the main vessel 6 are depicted, for example, in FIGS. 12D-12F. This arrangement is identical to that depicted in FIGS. 12A-12C, with the addition of a ramp 57 which is mounted on the balloon 54 and which provides a slight incline for the integrated stent-positioning guide wire 56A as it exits the positioning guide wire lumen 55A. As the guide wire 56A slides along the ramp 57, the distal portion 56B of the guide wire will move radially outwardly which helps position the guide wire and orient it into the side-branch vessel. In another arrangament for implanting the main-vessel stent in the main vessel, as depicted in FIGS.12G-12I, the guide wire lumen 55A passes underneath the main-vessel stent 20 and on top of the balloon 54. The distal end angled portion 55B curves along the balloon so that, as the distal end of the guide wire 56B advances out of the distal end angled portion 55B of the lumen, the distal end of the guide wire is traveling radially outwardly so that it can more easily locate and advance into the side-branch vessel 5.

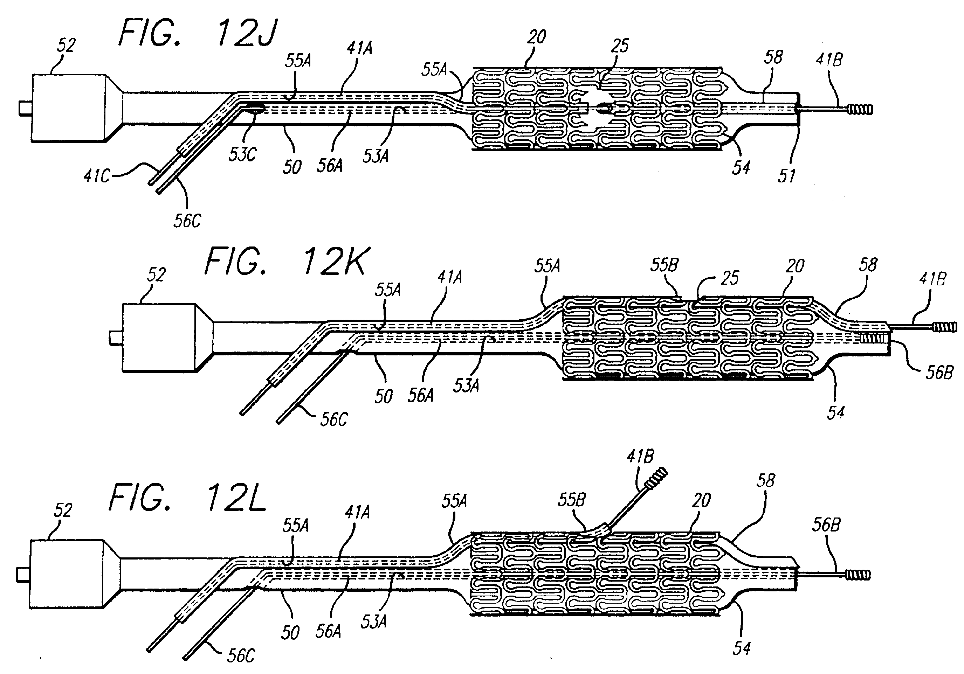

In still another arrangement for implanting the main-vessel stent 20 in the main-vessel 6, as depicted in FIGS. 12J-12L, the positioning guide wire lumen 55A is positioned under the stent 20 and terminates at the distal end angled portion 55B in the middle of the aperture 25. The distal end angled portion 55B of the guide wire lumen will spring outwardly, facilitating the advancement of the distal portion of the integrated stent positioning guide wire 56B into the side branch vessel. A distal guide wire lumen 58 is attached to the outer surface of balloon 54 and extends from the aperture 25 to essentially the distalmost end of the catheter.

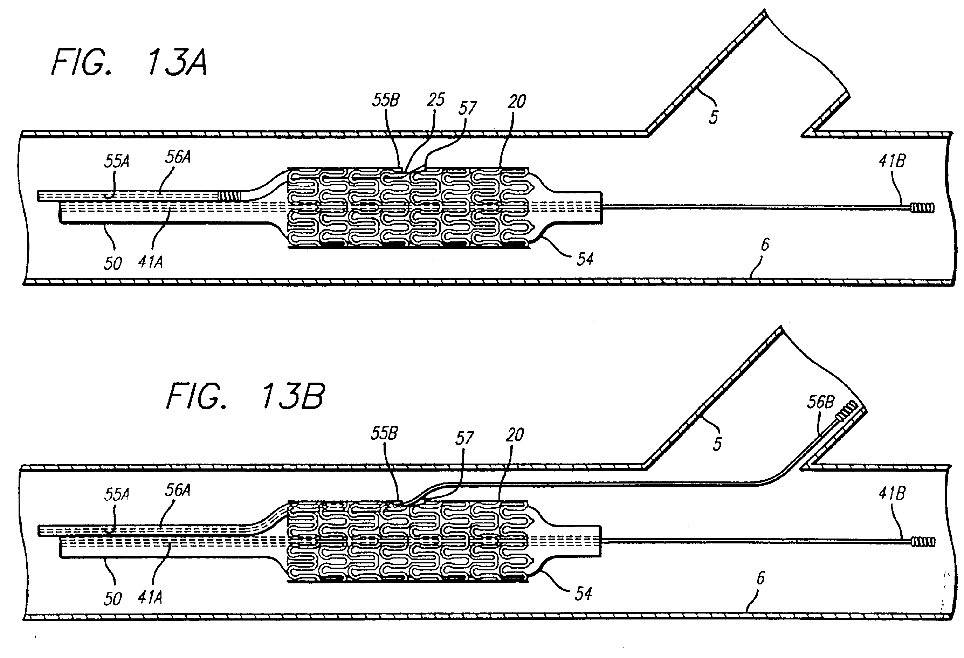

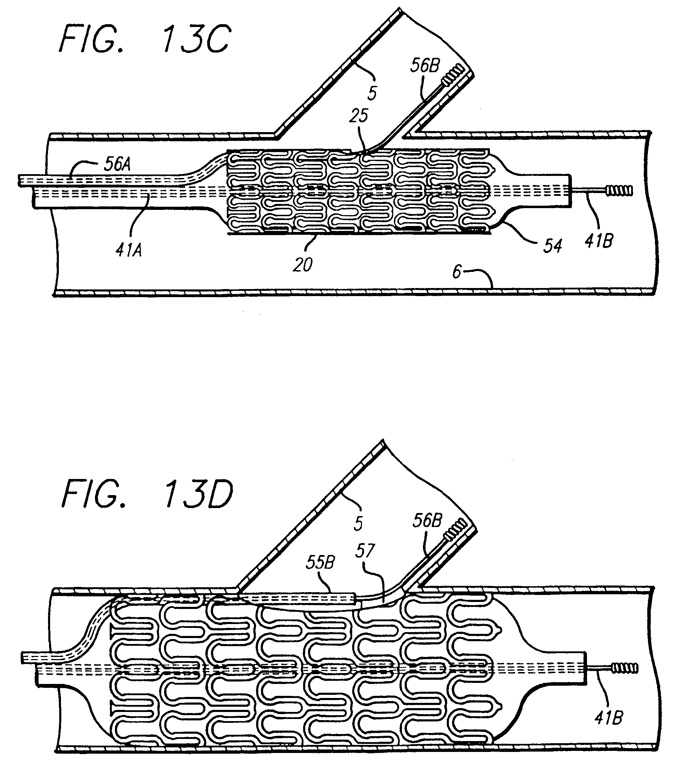

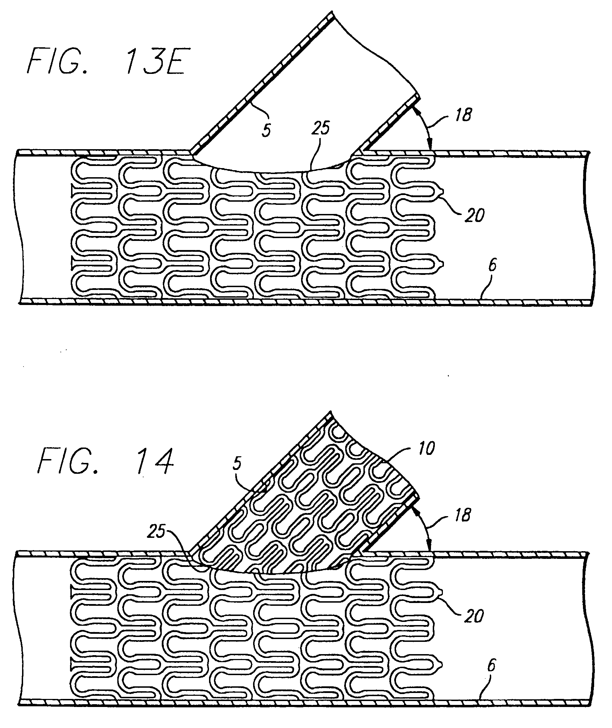

In one preferred arrangement for implanting the main-vessel stent 20 in the main-vessel 6, as depicted in FIGS. 12A-12I and 13A-13D, the integrated stent-positioning guide wire 41 A of the side-branch delivery assembly 41A remains in position in the main-vessel 6, while the side-branch tracking guide wire 3 6A is withdrawn from the patient. The main-vessel catheter 50 is back loaded onto the guide wire 41A by inserting the proximal end 41C of the wire into the distal end of the catheter and into the guide wire lumen 53A. The main-vessel catheter 50 is advanced over the guide wire 41 A and viewed under fluoroscopy until the main-vessel stent 20 is positioned in the main-vessel 6, just proximally of the side-branch vessel 5. The distal portion 56B of the integrated stent-positioning guide wire 56A of the main-vessel catheter then is advanced by the physician who pushes on the proximal portion 56C from outside the patient's body. The distal portion 56B of the guide wire 56A advances into and through the positioning guide wire lumen 55A, and passes underneath the proximal end of the main-vessel stent 20 and exits the angled portion 55B of the positioning guide wire of the lumen 56A and enters the side-branch vessel 5. The main-vessel catheter 50 then is advanced distally into the main vessel until resistance is felt, caused by the integrated stent-positioning guide wire of the main-vessel catheter 56A pushing up against the ostium of the side-branch vessel. The stiffness of the stent-positioning guide wire 56A causes the main-vessel catheter 50, with the main-vessel stent 20 thereon, to rotate, so that the aperture 25 faces the ostium of the side-branch vessel 5 where the proximal-angled stent 10 already is implanted.

The expandable member 54, which typically is a non-distensible expandable balloon, is inflated, thereby expanding the main-vessel stent 20 into contact with the main-vessel 6. The aperture 25 correspondingly expands and when properly aligned, provides a blood flow path between the aperture 25 and the proximal-angled stent 10 implanted in the side-branch vessel 5. As can be seen in FIGS. 12A-12I and 13A-13D, the positioning guide wire lumen 55A is positioned on the expandable member 54, such that when the expandable member is inflated, the positioning guide wire lumen 55A does not interfere with the implanting of the main-vessel stent 20. After the main-vessel stent is implanted in the main vessel, the expandable member 54 is deflated, and the main-vessel catheter 50 is withdrawn from the patient. As seen in FIG. 14, the bifurcated vessel has been fully covered by the stents, the side-branch vessel 5 being covered by the proximal-angled stent 10, and the main-vessel 6 being covered by the main-vessel stent 20, so that no portion of the bifurcation 4 is left uncovered and there is no overlap in the implanted stents.

In an alternative method of implanting the main-vessel stent 20 in the main-vessel 6 as depicted in FIGS. 12J-12L, the integrated stent-positioning guide wire 41 A of the side-branch delivery assembly, which here serves as the tracking guide wire, is advanced through the positioning guide wire lumen 55A and the distal guide wire lumen 58 attached to the balloon so that the guide wire advances distally of the distal end 51 of the main-vessel catheter. Thus, the guide wire distal end 41 B is advanced into the main vessel so that it is distal of the side-branch vessel 5. The integrated stent-positioning guide wire 56A, which until this point has remained within the guide wire lumen 53A (see FIG. 12K), is advanced distally as depicted in FIG. 12L and is advanced into the main vessel distally of the side-branch vessel. The guide wire 41 A then is withdrawn proximally through the guide wire lumen 58 until guide wire distal end 41B is able to exit the guide wire lumen distal end angled portion 55B, as shown in FIG. 12L. Because the angled portion of the positioning guide wire lumen 55B is preformed and has bias, it will spring outwardly. The guide wire 41A then can be advanced into the side-branch vessel for further positioning. As the catheter 50 is advanced over the guide wires, the distal portion 41 B of the guide wire will push against the ostium of the side-branch vessel thereby insuring the location of the main-vessel stent 20 and, importantly, the aperture 25 will align with the opening to the side-branch vessel 5.

A non-angulated stent can be implanted using the catheter system of FIGS. 7A-11D for stenting a side-branch vessel having an origin approaching ninety degrees (90°) in its takeoff from the main vessel. In this circumstance the positioning wire serves solely to arrest the forward movement of the stent precisely at the origin of the vessel for more precise positioning. However, the acute angle 18 is appropriate for a bifurcated vessel 4 in which the angulation is the acute angle 18, or less than ninety degrees (90°), Thus, consideration could be given to standard thirty degree (30°), forfy-five degree (45 °), and sixty degree (60°) angled stent designs for the proximal-angled stent 10, which should provide sufficient luminal wall coverage. The proximal-angled stent 10 has a wide range of applicability and can be used for stenting ostial side-branch lesions, ostial circumflex or left anterior descending (LAD) lesions where the bifurcation is an acute angle, or less than ninety degrees (90°), and ostial lesions involving the angulated origin of aright coronary or vein graft. Inportantly, the stents provide full coverage of the ostial intima without protruding into the main vessel and without compromising subsequent access to the distal portion of the main vessel.

In order to assist in properly aligning both the proximal-angled stent 10 and the main-vessel stent 20 in the side-branch vessel 5 and the main-vessel 6, respectively, the positioning guide wire lumen 39A, on the side-branch catheter 31, and the positioning guide wire lumen 55A, on the main-vessel catheter 50, can be radiopaque, or can have a radiopaque marker associated therewith so that each is visible under fluoroscopy. Thus, when advancing the side-branch catheter 31 and the main-vessel catheter 50, the proper orientation can be determined more easily by viewing the position of the positioning guide wire lumen 39A in connection with the main-vessel 6 or the positioning guide wire lumen 55A in connection with aligning the aperture 25 with the side-branch vessel 5. Additionally, the positioning guide wire 56A for positioning the main-vessel stent 20 and the positioning guide wire 41A for positioning the angled stent 10 either are radiopaque or have radiopaque portions, such as gold markers, to assist in positioning and orienting the catheters and stents during deployment and implantation.

While the foregoing description includes implanting the proximal-angled stent 10 in the side-branch vessel 5 prior to implanting the main-vessel stent 20 in the main-vessel 6, in an alternative arrangement, the order of the implanting procedures can be reversed. However, it should be understood that by implanting the main-vessel stent 20 in the main-vessel 6, and subsequently by implanting the proximal-angled stent 10 in the side-branch vessel 5, the aperture 25 must be carefully aligned with the side-branch vessel 5 so that the side-branch catheter 31 can be advanced through the expanded main-vessel stent 20 and the aperture 25 and into the side-branch vessel 5 for implanting the proximal-angled stent 10.

While the side-branch catheter 31 and the main-vessel catheter 50 have been described herein as being of the rapid-exchange type, they also can be of a conventional over-the-wire-type catheter. In over-the-wire-type catheters, the guide wire lumen extends from the distal end of the catheter to the proximal end with no side port as is found in the rapid-exchange-type catheters. Typical of over-the-wire-type catheters is the type disclosed in U.S. Patent Nos. 4,323,071 and B1 4,323,071, commonly assigned and commonly owned by Advanced Cardiovascular Systems, Inc., Santa Clara, California.

While the invention herein has been illustrated and described in terms of an assembly for stenting bifurcated vessels, it will be apparent to those skilled in the art that the stents and delivery systems herein can be used in the coronary arteries, veins and other arteries throughout the patient's vascular system. Certain dimensions and materials of manufacture have been described herein, and can be modified without departing from the scope of the invention as defined in the appended claims.

## Claims

1. An assembly for treating a bifurcation, the assembly comprising:
a first, side branch catheter (31) having a balloon portion;
a proximal-angled stent (10) mounted on the balloon portion of the first, side-branch catheter (31) for implanting in a side-branch vessel (5) adjacent a bifurcation (4) between the side-branch vessel (5) and a main vessel (6), the proximal-angled stent (10) comprising a cylindrical member (11) having a longitudinal axis (12), the cylindrical member having a distal end (13) and a proximal end (14), the distal end (13) forming a first plane section (15) substantially transverse to the longitudinal axis, and the proximal end (14) forming a second plane section (16) having an acute angle (18) relative to the longitudinal axis (12);
a second, main-vessel catheter (50) having a balloon portion; and
a main-vessel stent (20) mounted on the balloon portion of the second, main-vessel catheter (50) for implanting in the main vessel (6) adjacent the bifurcation (4), the main-vessel stent (20) comprising a cylindrical member (21) having a distal end (22), a proximal end (23), an outer wall surface (24) therebetween, and an aperture (25) in the outer wall surface (24), the aperture (25) being configured so that, upon expansion, it approximates the diameter of the expanded proximal end (14) of the proximal-angled stent (10).

2. The assembly of claim 1, wherein the proximal-angled stent (10) is expandable from a first smaller diameter for delivery in a body lumen to a second expanded diameter by plastically deforming the stent (10) beyond the elastic limits of the material forming the stent (10).

3. The assembly of claim 1, wherein the proximal-angled stent (10) is formed from a self-expanding material so that the stent (10) expands from a first smaller diameter for delivery through a body lumen to a second implanted diameter in the body lumen.

4. The assembly of claim 1, wherein the main-vessel stent (20) is expandable from a first smaller diameter for delivery in a body lumen to a second expanded diameter by plastically deforming the stent (20) beyond the elastic limits of the material forming the stent (20).

5. The assembly of claim 1, wherein the main-vessel (20) stent is formed from a self-expanding material so that the stent (20) expands from a first smaller diameter for delivery through a body lumen to a second implanted diameter in the body lumen.

## Patentansprüche

1. Vorrichtung zur Behandlung einer Verzweigung, wobei die Vorrichtung Folgendes umfasst:
einen ersten Abzweigungskatheter (31), der einen Ballonabschnitt aufweist;
einen proximal abgewinkelten Stent (10), der auf dem Ballonabschnitt des ersten Abzweigungskatheters (31) befestigt ist, zum Einsetzen in ein Abzweigungsgefäß (5) benachbart zu einer Verzweigung (4) zwischen dem Abzweigungsgefäß (5) und einem Hauptgefäß (6), wobei der proximal abgewinkelte Stent (10) ein zylindrisches Element (11) mit einer Längsachse (12) aufweist, wobei das zylindrische Element ein distales Ende (13) und ein proximales Ende (14) aufweist, wobei das distale Ende (13) eine erste Schnittebene (15) im Wesentlichen quer zur Längsachse bildet und das proximale Ende (14) eine zweite Schnittebene (16) mit einem spitzen Winkel (18) relativ zur Längsachse (12) bildet;
einen zweiten Hauptgefäßkatheter (50), der einen Ballonabschnitt aufweist; und
einen Hauptgefäßstent (20), der auf dem Ballonabschnitt des zweiten Hauptgefäßkatheters (50) befestigt ist, zum Einsetzen in das Hauptgefäß (6) benachbart zu der Verzweigung (4), wobei der Hauptgefäßstent (20) ein zylindrisches Element (21) aufweist, das ein distales Ende (22), ein proximales Ende (23), eine äußere Wandfläche (24) dazwischen und eine Öffnung (25) in der äußeren Wandfläche (24) aufweist, wobei die Öffnung (25) so ausgestaltet ist, dass sie sich bei Ausdehnung an den Durchmesser des ausgedehnten proximalen Endes (14) des proximal abgewinkelten Stents (10) angleicht.

2. Vorrichtung nach Anspruch 1, wobei der proximal abgewinkelte Stent (10) von einem ersten kleineren Durchmesser zum Einbringen in ein Körperlumen auf einen zweiten ausgedehnten Durchmesser durch plastische Verformung des Stents (10) über die Elastizitätsgrenzen des den Stent (10) bildenden Materials hinaus ausdehnbar ist.

3. Vorrichtung nach Anspruch 1, wobei der proximal abgewinkelte Stent (10) aus einem selbst-ausdehnenden Material gebildet ist, so dass sich der Stent (10) von einem ersten kleineren Durchmesser zum Einbringen durch ein Körperlumen auf einen zweiten eingesetzten Durchmesser in dem Körperlumen ausdehnt.

4. Vorrichtung nach Anspruch 1, wobei der Hauptgefäßstent (20) von einem ersten kleineren Durchmesser zum Einbringen in ein Körperlumen auf einen zweiten ausgedehnten Durchmesser durch plastische Verformung des Stents (20) über die Elastizitätsgrenzen des den Stent (20) bildenden Materials hinaus ausdehnbar ist.

5. Vorrichtung nach Anspruch 1, wobei der Hauptgefäßstent (20) aus einem selbstausdehnbaren Material gebildet ist, so dass sich der Stent (20) von einem ersten kleineren Durchmesser zum Einbringen durch ein Körperlumen auf einen zweiten eingesetzten Durchmesser in dem Körperlumen ausdehnt.

## Revendications

1. Ensemble pour le traitement d'une bifurcation, l'ensemble comprenant :
un premier cathéter de branchement latéral (31) ayant une portion de ballonnet ;
un stent angulaire proximal (10) monté sur la portion de ballonnet du premier cathéter de branchement latéral (31) pur l'implantation dans un vaisseau de branchement latéral (5) adjacent à une bifurcation (4) entre le vaisseau de branchement latéral (5) et un vaisseau principal (6), le stent angulaire proximal (10) comprenant un élément cylindrique (11) ayant un axe longitudinal (12), l'élément cylindrique ayant une extrémité distale (13) et une extrémité proximale (14), l'extrémité distale (13) formant une première section plane (15) sensiblement transversale à l'axe longitudinal, et l'extrémité proximale (14) formant une seconde section plane (16) ayant un angle aigu (18) relativement à l'axe longitudinal (12) ;
un deuxième cathéter de vaisseau principal (50) ayant une portion de ballonnet ; et
un stent de vaisseau principal (20) monté sur la portion de ballonnet du deuxième cathéter de vaisseau principal (50) pour l'implantation dans le vaisseau principal (6) adjacent à la bifurcation (4), le stent de vaisseau principal (20) comprenant un élément cylindrique (21) ayant une extrémité distale (22), une extrémité proximale (23), une surface de paroi extérieure (24) entre celles-ci, et une ouverture (25) dans la surface de paroi extérieure (24), l'ouverture (25) étant configurée de telle sorte que, lors de l'expansion, elle s'approche du diamètre de l'extrémité proximale expansée (14) du stent angulaire proximal (10).

2. Ensemble selon la revendication 1, dans lequel le stent angulaire proximal (10) peut se dilater d'un premier diamètre plus petit pour la délivrance dans une lumière corporelle à un deuxième diamètre expansé par la déformation plastique du stent (10) au-delà des limites élastiques du matériau formant le stent (10).

3. Ensemble selon la revendication 1, dans lequel le stent angulaire proximal (10) est réalisé à partir d'un matériau auto-expansible de telle sorte que le stent (10) se dilate d'un premier plus petit diamètre pour la délivrance à travers une lumière corporelle à un deuxième diamètre implanté dans la lumière corporelle.

4. Ensemble selon la revendication 1, dans lequel le stent de vaisseau principal (20) peut se dilater d'un premier plus petit diamètre pour la délivrance dans une lumière corporelle à un second diamètre expansé par la déformation plastique du stent (20) au-delà des limites élastiques du matériau formant le stent (20).

5. Ensemble selon la revendication 1, dans lequel le stent de vaisseau principal (20) est formé à partir d'un matériau à auto-expansion de telle sorte que le stent (20) se dilate d'un premier plus petit diamètre pour la délivrance à travers une lumière corporelle à un second diamètre implanté dans la lumière corporelle.
